# EUROPEAN PATENT SPECIFICATION

(11) **EP 1 762 202 B1**
(45) Date of publication and mention of the grant of the patent: **05.01.2011**
(21) Application number: 06017261.6
(22) Date of filing: 27.08.1998
(51) Int. Cl.: A61F 2/44

(54) **Cortical bone cervical Smith-Robinson fusion implant**
Kortikalknochenimplantat für Wirbelfusion nach Smith-Robinson
Implant de substance corticale pour des procédés de fusion des vertèbres cervicales de Smith-Robinson

(30) Priority: 27.08.1997 US 920630
(43) Date of publication of application: 14.03.2007
(62) Divisional of application: 98941086.5
(73) Proprietor: RTI Biologics, Inc., Alachua, FL 32615 (US); Medtronic Sofamor Danek, Inc., Memphis, TN 38132 (US)
(72) Inventor: Carter, Kevin C., Alachua, FL 32615 (US); Sander, Tom, Alachua, FL 32615 (US)
(74) Representative: Samuels, Lucy Alice

(56) References cited:
- EP-A2- 0 307 241
- EP-A2- 0 517 030
- WO-A-97/15248
- WO-A2-97/14378
- FR-A1- 2 724 312
- US-A- 5 397 364

## Description

### 1.0 Background of the Invention

### 1.1 Field of the Invention:

This invention relates to a cortical bone implant for use in cervical Smith-Robinson vertebral fusion procedures.

### 1.2 Background Art:

Since at least the mid to late 1950's anterior cervical spinal fusions have been performed in order to alleviate chronic neck, arm and shoulder pain caused by trauma, disc herniation, or spondylosis (Robinson and Smith, 1955; Smith and Robinson, 1958). The classic procedure referred to as the Smith-Robinson cervical fusion employs a horseshoe-shaped graft to promote vertebral fusion (Robinson *et al.,* 1962). The Cloward technique employs a Cancellous bone dowel (Cloward, 1958), and the Bailey-Badgley procedure uses a strut (Bailey and Badgley, 1960). In a study comparing the compressive load capacity of the various implants used according to these procedures, it was found that the Smith-Robinson graft could sustain loads up to 344 N, the Cloward dowel could sustain loads of up to 188 N, and the Bailey-Badgley type could sustain loads up to 195 N, (White and Hirsch, 1972). In a modified Smith-Robinson procedure, the horseshoe-shaped implant is inserted with the cortical end of the implant located posteriorly, which has been reported to increase the fusion rate while decreasing the graft extrusion and collapse sometimes experienced with the Cloward dowels (Whitecloud and Dunsker, 1993). However, in a recent study evaluating the success and relief rates achieved according to these procedures, it was found that less than 100% success rate (fusion, patient improvement and absence of complications) was achieved, regardless of which method or implant was used (Grooms *et al*, 1996).

U.S. Patent No. 5,306,309, discloses a spinal disk implant comprising a solid body of biocompatible synthetic material arranged to define a right-rectangular solid having two opposed side faces and two opposed transverse faces, including a convexly curved anterior face and a posterior face, for implantation in the intervertebral space. The discussion of vertebral and intervertebral morphology is hereby incorporated by reference.

U.S. Patent No. 5,609,635, discloses a lordotic interbody spinal fusion implant comprising a wedge shaped metallic cage for insertion into the intervertebral space.

U.S. Patent No. 5,306,307, discloses a ceramic spinal disk implant having a serrated edge. The present invention addresses the need in the art for improvements to both the implant and the avoidance of post-surgical complications from anterior cervical fusions. The present invention provides a new cortical bone implant for use in achieving anterior cervical fusions when implanted according to the Smith-Robinson procedure.

### 2.0 Summary of the Invention

An implant composed substantially of cortical bone is provided according to claim 1, for use in cervical-Smith-Robinson vertebral fusion procedures. The implant is preferably derived from allograft or autograft cortical bone sources, is machined to form a substantially half "D"-shaped implant having a canal into which osteogenic, osteoinductive, or osteoconductive material may be packed. The implant is inserted into the space between adjacent cervical vertebrae to provide support and induce fusion of the adjacent vertebrae.

### 3.0 Brief Description of the Drawings

Figure 1 provides several views of a fusion implant not in accordance with claim 1.
Figure 2 provides views of a core cutter and drill assembly and the bone plug formed by cutting into the diaphysis of a long bone when such a core cutter and drill assembly is used.
Figure 3 provides a view of broach and an asymmetric canal formed by use of such a broach.
Figure 4 provides several views of an apparatus for machining a profile on the exterior surface of an implant.
Figure 5 provides a view of an apparatus for inscribing retention teeth in the upper surface, lower surface or both upper and lower surfaces of an implant.
Figure 6 provides several views and dimensions for specific embodiments of an implant not within the claims.
Figure 7 provides a view of a stacked embodiment of an implant not within the claims.
Figure 8 provides several views of an implant of this invention formed by juxtaposition of mirror image halves of the implant, as well as an embodiment useful for posterior lumbar intervertebral fusion procedures (PLIFs).
Figure 9 provides a view of a stacked embodiment of an implant not within the claims wherein the stacked constituents thereof are retained in registered relationship by press-fitting or otherwise bringing more than one implant into contact with each other and having a cancellous plug or other biocompatible material located in the central canal of each stacked implant, thereby acting as a retention pin.
Figure 10 shows an alternate method for producing bone stock for making an implant of essentially unlimited height from the anterior margin of the tibia or the linea aspera of the femur.
Figure 11 shows dimensions and further processing of an implant of an implant produced according to the alternate method of figure 10.
Figures 12-17 show final profiles for implants produced according to the alternate method of figures 10 and 11.

### 4.0 Detailed Description of the Invention

According to this invention, a substantially half "D"-shaped cortical bone implant for cervical Smith-Robinson fusions is produced, preferably under aseptic conditions. Class 10 clean room processing is desirable, and sterilization of all machining tools is likewise preferred, (particularly after switching from one allograft donor to the next), so that the finished product may be treated by standard techniques known in the art (alcohol, peroxide, or like treatments), prior to storage and shipment to physicians for use in implantation procedures. Because of the peculiarities of working with bone, and in particular, because of the desirability of maintaining aseptic conditions while working with this material, novel approaches have been adopted in the production of the product of this invention.

The implant is preferably formed from cortical bone obtained from tibia, femur or other source of strong cortical bone. The bone source may be autograft or, due to possible complications at the donor site (infection, pain, delayed healing), is preferably, allograft bone. In addition, it is critical that the source bone be derived from a donor whose medical history is well known (absence of transmissible diseases, cancer, osteoporosis), and that the donor bone be obtained under aseptic conditions according to accepted practices in the art of tissue banking. In addition, extensive *in vitro* testing should be conducted to ensure the absence of pathogenic agents.

The approach adopted in describing the implant of this invention is to first provide a narrative disclosure of preferred methods for making the implant, followed by a detailed description of the implant itself, followed by a detailed description of various apparatuses and aspects of the machining process, and finally, a detailed description of the method of using the implant.

### 4.1 Narrative Description of Implant Manufacture

While any shape of cortical bone may be used to begin with, we have found that for consistent production of cortical bone which may be reliably machined, it is advantageous to commence with a plug of bone which extends from the exterior of the diaphysis of a long bone toward the intramedullary canal (where, *in vivo*, the bone marrow resides). The result is a bone plug or dowel which has an outer substantially cortical end and an internal end which is composed largely of soft cancellous bone. In cutting the bone plug, we have discovered that the use of a core cutter is convenient. This device comprises an outer coring element of any desired diameter, whereby the diameter of the bone plug is defined, and a centrally located solid drill bit, which provides a canal through the center of the bone plug as well as stability for the core cutting element The core cutter-drill assembly is preferably torqued by an air drill, driven by sterile air, and the source bone is preferably immobilized in a sterilized vice during the core-cutting process.

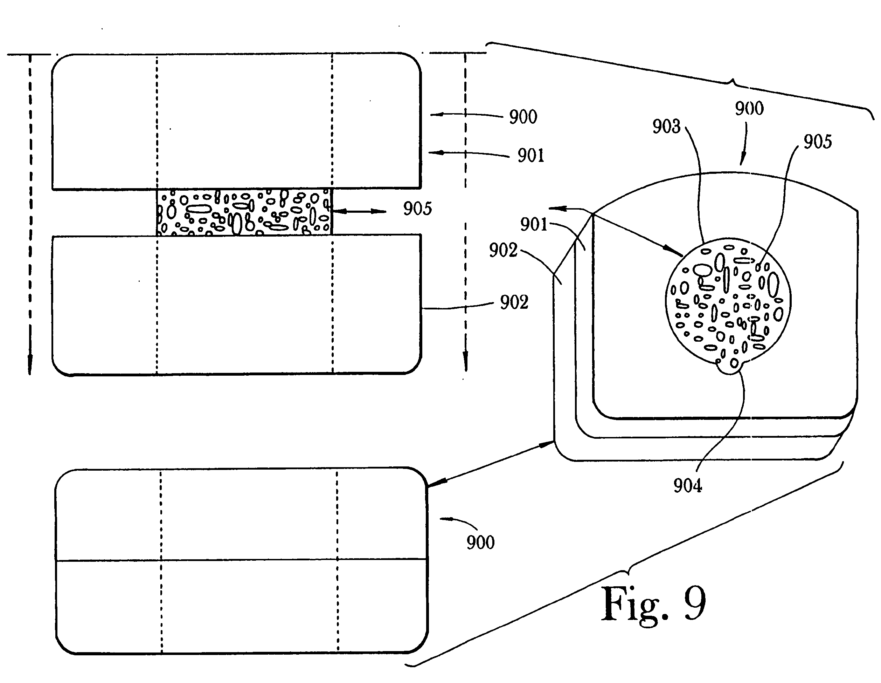

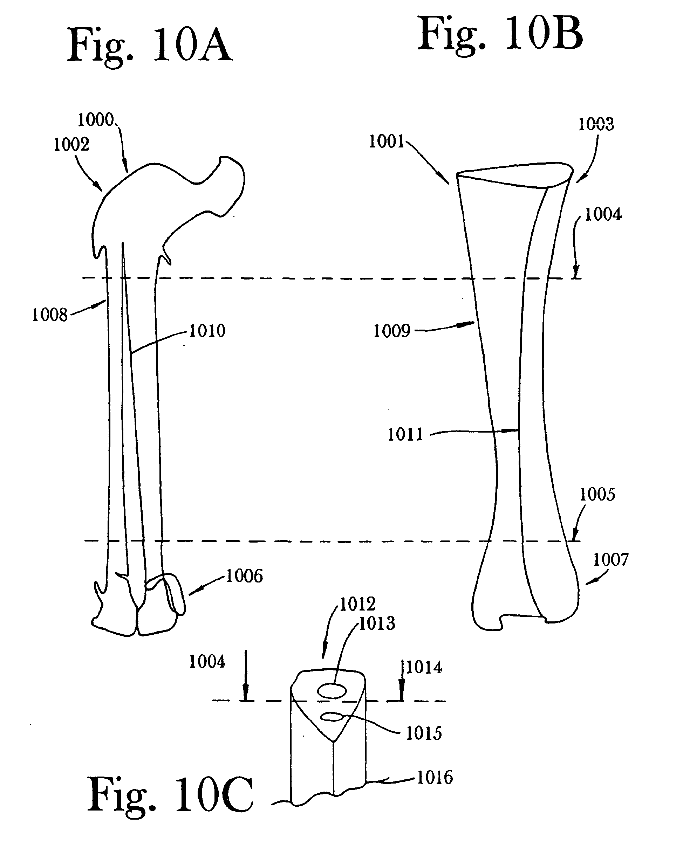

We have discovered that in the above-described manner, cortical bone implants may be fashioned having heights, widths and lengths which are practically useful in the Smith-Robinson cervical fusion method According to this method, the height of the implant is only limited by the distance from the exterior of the bone diaphysis to the intramedullary canal. However, we have discovered that, by this method, final implant heights from about 7 mm to about 14 mm may be produced, depending on the choice of bone source and the location on the bone from which the bone plug is cored. Since it is extremely rare for the cervical intervertebral space to extend beyond these limits, this method is therefore capable of supplying implants of required or useful heights. Likewise, the length and width of the implant are defined by the diameter of the core-cutter, and final lengths and widths of between about 7 and 14 mm are easily provided for by this method. In addition, where the need arises for heights between about 10 mm and 14 mm, or if difficulty is experienced in obtaining donor bone having a sufficient width from the exterior of the bone to the intra-medullary canal to provide such heights, alternate methods of producing the implant of desired heights disclosed herein may be employed. For example, in a first such alternate method, implants of this invention are produced and then stacked to provide a unitary implant of the desired height dimensions. Such stacked implants may be maintained in a unitary association by drilling appropriate holes through the height of the implant, and inserting therein appropriate retention pins made from any desirable material, including cortical bone, bioabsorbable synthetic polymer, titanium or other metallic retention pins. Alternatively, the stacked implants may be retained in a unitary association by means of a plug of cancellous bone, hydroxyapatite or other biocompatible, osteoconductive or osteoinductive material, and press-fitting the stacked implants to achieve the desired height (see figure 9). In a further alternate method, a section of cortical bone along the long axis of a long bone may be machined according to methods known in the art. By then further shaping and cutting appropriate heights in such cortical bone, and bringing halves of the implant into juxtaposition with each other, implants of any desired shape and height are produced. In yet a further alternate procedure, (see figures 10-17), unitary implants of this invention of essentially unlimited height are produced by lengthwise sectioning the anterior margin of the tibia or linea aspera of the femur, segmenting the substantially triangular cortical bone to desired heights, drilling a cannulation through the segments thus produced, and finally shaping the implants to desired dimensions, as defined below for the first principal method of making the implant of this invention.

Continuing with a description of the first method for making the implant of this invention, the cancellous bone on the internal side of the bone plug is removed by any convenient means, including with a saw, an abrasive means such as a diamond tipped rotary sander, or a tooling bit mounted in a lathe, to produce a "washer" shaped piece of substantially cortical bone. Both the internal and external ends of the bone plug should be machined flat, thereby forming a top face and a bottom face, each of which is substantially planar, and preferably parallel. While the cancellous bone is partially or completely removed by this process, there remains a slight difference in the density of the bone from the external (cortical) to the internal (cancellous or originally intra-medullary) aspect of the bone plug. It is desirable to record the orientation of the bone plug as subsequent machining steps proceed most efficiently when machined from the external aspect toward the internal aspect.

Once the external profile has been machined, the implant is removed from the spindle, and the machining of the implant may either be terminated, or an external feature may be machined into the upper and lower surfaces to prevent backing out of the implant upon insertion into the intervertebral space. This may be achieved by a number of means, such as by machining annular rings, indentations and projections, ribbing or teeth into the upper, lower, or both surfaces of the implant. In a preferred embodiment of this invention, the implant is passed through a set of opposing jaws bearing teeth which broach a tooth-shaped profile into the implant as it is forced through the jaws. Alternatively, the implant is passed several times over a ridged surface which cuts the desired tooth profile into the upper, lower or both surfaces of the implant. Preferably, the thus formed teeth angle toward the anterior (convexly curved) face of the implant to prevent backing out of the implant once it is inserted into an appropriately shaped cavity formed in the intervertebral space in an anterior aspect of the cervical spine. In order to accommodate the difficulty surgeons experience in forming precise angles when forming such cavities in the spine, (see for example U.S. patent No. 5,397,364 disclosing a beveled edge to reduce trauma upon insertion of a metallic spinal implant), a beveled edge of defined radius is preferably machined into three faces of the implant, but leaving the anterior face unbeveled. The sharp anterior edge, like the teeth in the upper and lower surfaces of the implant, retards backing out of the implant.

### 4.2 Detailed Description of Implants

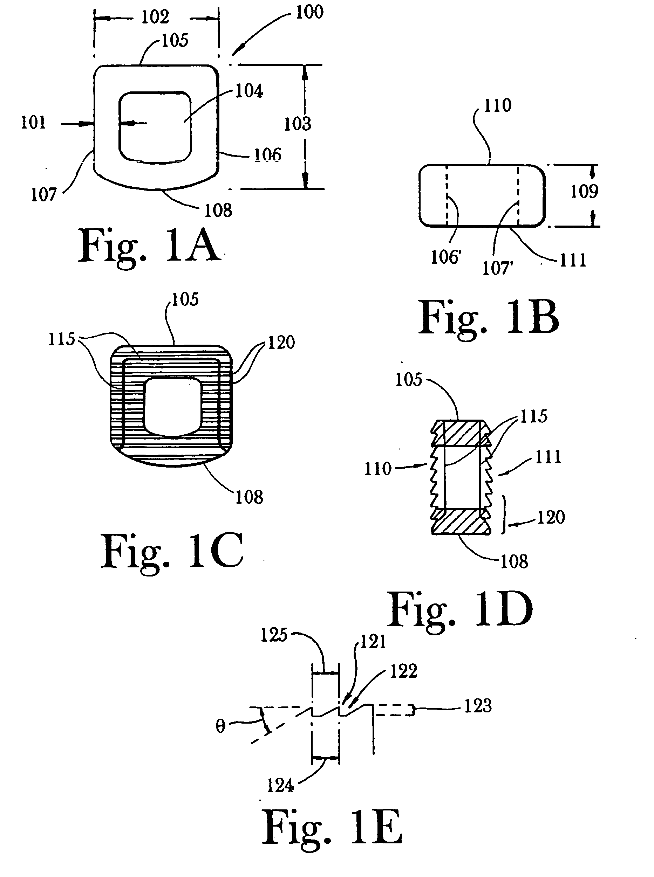

Referring now to figure 1A, there is shown a top view, as if viewed from the top of the spinal column, of a substantially "D"-shaped cortical bone implant **100.** The implant has a wall thickness **101,** a length **103,** a width **102,** and an internal canal **104**, which fall within desired tolerances (see discussion below). The implant comprises four contiguous walls, including a substantially straight rear wall **105,** substantially straight side walls **106** and **107,** and a preferably curved front wall **108.** In figure 1B, there is shown a side view of the implant 100, revealing the height **109,** of the implant. In addition, this view shows, in outline, the internal side walls **106'** and **107'** of the internal canal, **104.** It also shows the top **110** and bottom **111** surfaces of the implant. In figure 1C, there is shown a top view of an embodiment of the implant 100 in which an external feature **120** has been inscribed onto the top **110** and bottom **111** surfaces of the implant. In addition, a "radius" or bevel **115** is shown on the two side and posterior edges of the implant. Figure 1D shows a side view of the implant **100** in which the inscribed feature **120** can clearly be seen in the top **110** and bottom **111** surfaces of the implant. In this view, it can be seen that the external feature **120** has the side profile of a set of teeth, all of which angle toward the anterior face **108** of the implant. An outline of the bevel **115** is also evident in this view, as is the rounded posterior edge **105.** As can be seen, in this embodiment of the invention, the anterior edge **108** is maintained with a sharp edged. In figure 1E, there is shown a detail of one embodiment of the inscribed feature **120** on the portion of the implant indicated in figure 1D. In a preferred embodiment, the feature **120** defines a tooth-like structure, with teeth **121** separated from each other by concavities **122.** An angle θ defines the grade of the concavity as it ramps to the tooth. The tooth height **123,** space between teeth **124,** and aperture of the concavity **125** may all be defined by the manufacturer to optimize retention of the implant within the cervical spine after proper placement.

### 4.3 Detailed Description of the Method of Manufacturing Implants

Because of the peculiar nature of bone, and the desirability of sterile or aseptic manufacturing, specific and specialized procedures and apparatuses are required for successful formation of the implants of this invention. Those skilled in the art will recognize that, based on the methods and apparatuses disclosed herein, the implant of this invention may be manufactured by alternate means suggested by those described herein. Nonetheless, through careful design and knowledge of bone structure, instruments for the manufacture of the implant of this invention have been invented for this purpose. In what follows, specific details with respect to preferred method and apparatuses for making the implant of this invention are provided. It should be recognized that the invention should not be construed as being limited to these specifics.

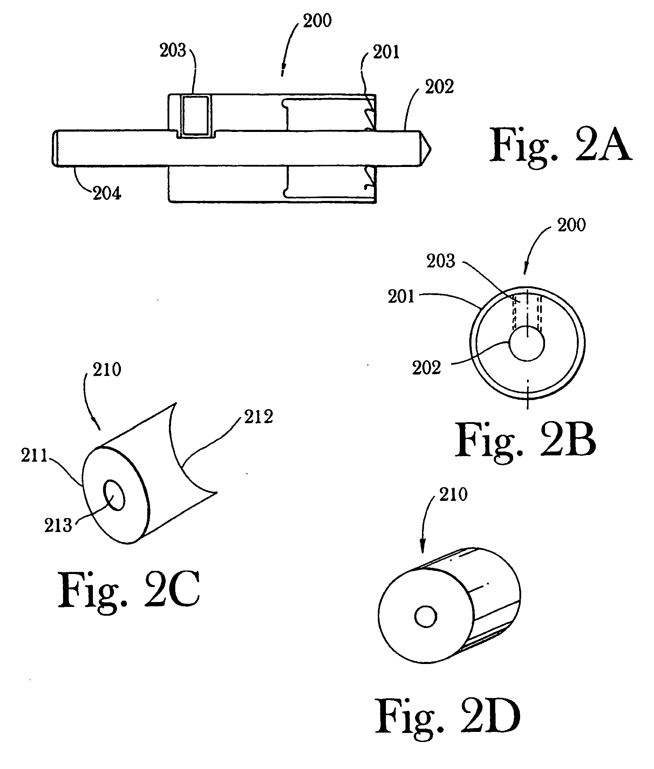

Referring to figure 2, there is shown in side view in figure **2A** a core cutter **200,** having a core bit **201** which is affixed by a set screw **203** to the shaft **204** of a drill bit **202,** centrally located within and coaxial with the core cutter. In figure 2B, an end-on view of the core cutter **200** is provided showing the set screw **203** in outline. Figure 2C shows a side view of the bone plug **210** which is formed by cutting a plug of bone from the diaphysis of a long bone using the core cutter **200.** At one end, **211,** originally the external cortical surface of the bone shaft, there is a substantially cortical bone surface through which a hole **213** is formed by the central bit **202** of the core cutter **200.** The other end, **212,** is an irregular and bone surface which, *in vivo*, formed part of the wall of the intramedullary canal. Cancellous bone or other microstructure at the end **212** is removed, and both ends are ground, cut or otherwise machined to be substantially flat and parallel, to form the substantially cortical bone plug **210** shown in figure 2D.

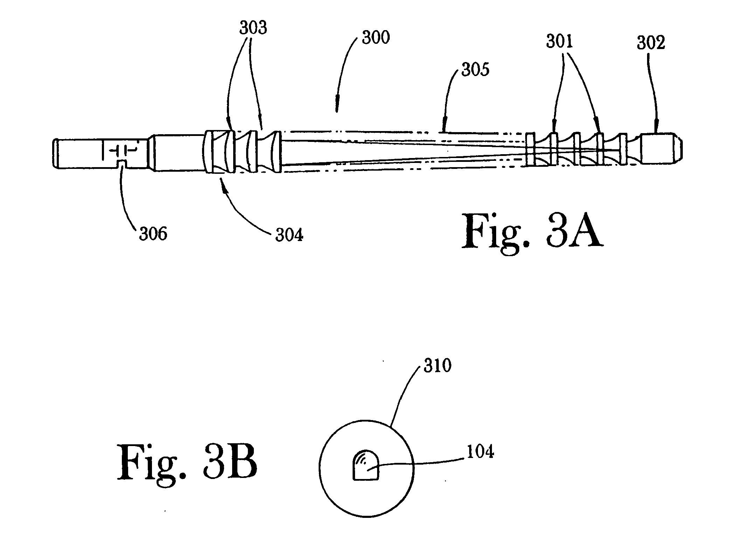

Referring to figure 3, there is shown in figure 3A an internal canal profile broaching tool **300.** A plurality of spaced-apart ribs or rings **301** are provided along the length of the broach which taper from a substantially circular shape at the insertion end **302** of the broach, to substantially "D"-shaped rings **303** (or any other desired shape) at the completion end **304** of the broach **300** (intermediate ribs **305** are not shown; rather, the outline of the taper angle is shown). A notch or groove **306** is provided in the broach completion end **304** for releasably affixing the broach into a means, such as a press, for forcing the broach through the implant canal. In figure 3B, there is provided an end-on view of the cancellous bone plug **310** after the broaching procedure is completed. As can be seen, the internal canal **104** has been converted from a circular canal into a substantially "D"-shaped canal. As will be appreciated from this disclosure, any of a number of different asymmetric shapes in the internal canal **104** may be defined by this or analogous means, the principal goal being to provide a purchase (referred to herein as a "key way") within the implant for external machining of the implant. In one embodiment (see figure 9), the canal may be retained as a substantially circular canal, and a slot **904** is machined therein to provide the necessary asymmetry to form a key way.

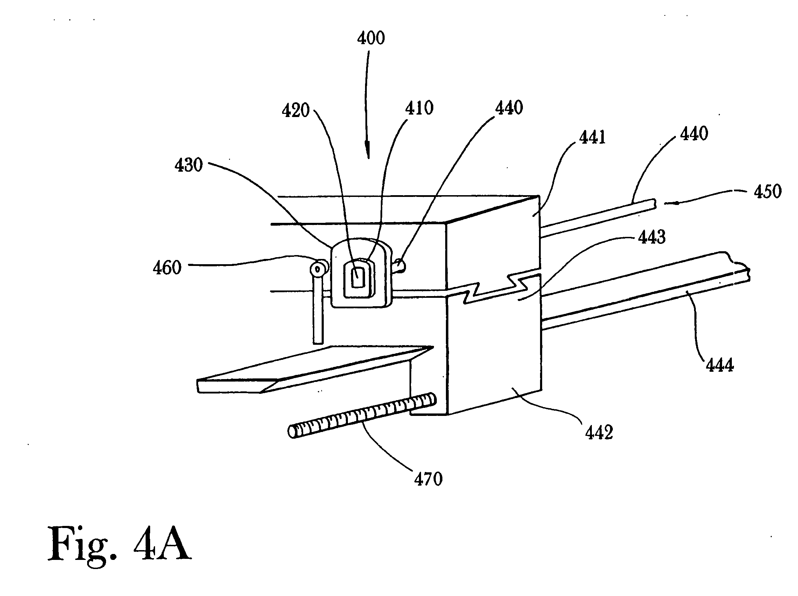

Having formed a key way within the implant, it is possible to modify the external profile of the implant. Referring to figure 4A, this is conveniently achieved by affixing the implant **410** to the spindle **420** of a lathe **400.** The spindle shaft **440** extends, through bearings (not shown), to a means **450** (such as a handle or a motor) for rotating the spindle. Affixed to the spindle-shaft is a cam **430,** the shape of which defines the ultimate external profile of the implant **410.** The spindle shaft **440** and bearings are mounted in a cross slide **441,** which translates in a first plane, referred to as the "Y-plane". Motion in the Y-plane is limited by contact of the cam **430** with a limiting means **460** such as a cam follower, which remains in register with a carriage **442** which translates along a plane, the "X-plane", transverse to the Y-plane motion of the cross-slide . The cross-slide is mounted in a slide-way **443** of the carriage **442,** which in turn is slideably mounted on the bed **444** of the lathe, such that the carriage **442** is permitted to translate along the X-plane. Travel of the slide **442** along the X-plane is limited by means of a stop screw **470.**

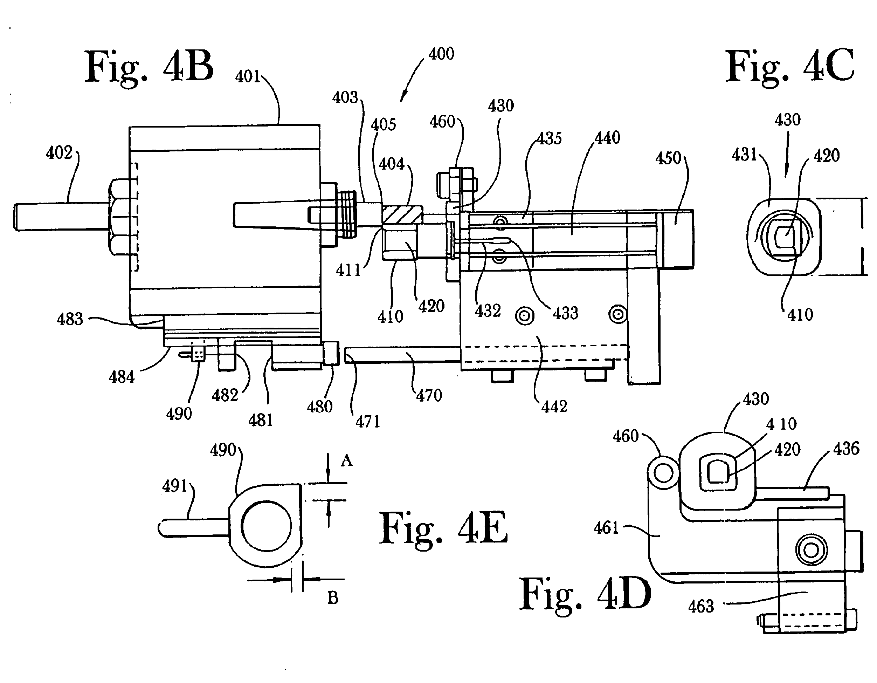

Further detail of this means for generating the external profile of the implant is provided in figure 4B, which provides a side view of one specific embodiment of the implant external profile generator **400.** An air driven turbine within housing **401** provides a source of torque to turn a shaft **402.** A means for cutting or grinding the external surface of the implant **410,** such as an appropriately fashioned cutter or bit having a non-cutting end **403** for fixation to the shaft **402.** Extending from the non-cutting end **403** which has a first diameter, is a cutting surface **404,** having a second, smaller diameter. A "shoulder" **405,** forms a radius extending between the smaller diameter of the cutting surface **404** and the larger diameter of non-cutting surface **403.** The cutting surface **404** is contacted with the implant blank **410,** mounted on spindle **420,** to which, as described above is mounted an asymmetric cam **430.** The thus mounted implant blank **410** is brought into contact with the cutting surface **404,** by virtue of translation in the X-plane of the carriage **442.** The spindle **420,** and thus the asymmetric cam **430** are rotated, manually or by motor driven means, through shaft **440** and handle **450** which are attached concentrically with the cam **430.** Preferably, the asymmetric cam **430** is elastically biased toward a stationary cam follower **460.** In this fashion, after several revolutions of the handle **450,** the shape of the asymmetric cam **430** generates the desired external profile of the implant **410** riding on the spindle **420,** through contact with the rotating cutting surface **404.**

To ensure that the implant blank is machined only up to the point that the forward edge **411** of the implant approaches but does not contact the "shoulder" **405** on the cutter, a stop screw **470** is provided. The stop screw **470** is set to prevent further advancement of the implant blank **410** by stopping advancement of the carriage **442** when the leading edge **471** of the stop screw comes into contact with a measuring screw **480.** The appropriate setting of the stop screw **470** is achieved at the start of the milling process by first placing the implant **410** between the end **481** of the measuring screw **480** and an anvil **482,** and tightening the measuring screw **480** until it just makes contact with the implant. In this fashion, the measuring screw **480** and anvil **482** essentially form a micrometer, with the gap being defined by the width of the implant. Both the measuring screw **480** and anvil **482** are housed within a measuring slide **483** which, when slid all the way to the left as shown in figure 4B, abuts a rotateable stop cam **490,** retained within the same slide-way as the measuring slide **483** by a retainer **484.** The rotateable stop cam **490** may be set in either of two positions, which produces a difference in the stopping point of the stop screw **470** of approximately 0.06". The significance of this difference is that the first position arrests advancement of the stop screw **470** (and therefore the carriage **442**) just before the implant **410** contacts the radius shoulder **405** of the cutting surface **404.** In the second position, the stop cam **490** allows the stop screw to advance the additional approximately 0.06" to allow contact of the implant **410** with the shoulder **405** of the cutting surface **404** to thereby bevel the edges of the implant **410** that are thus contacted. Accordingly, in the pre-milling setup, the stop cam **490** should be rotated such that the stop screw **470** is forced to stop the extra 0.06", following which a further processing step may be carried out in which the stop cam **490** is rotated to the second position in which the stop screw **470** is allowed to advance this additional approximately 0.06".

In figure 4C, there is provided an end-on, rear view (i.e. looking from the handle **450** toward the spindle **420**) of the asymmetric cam **430,** the spindle **420** and the implant **410.** In addition, in this detail view, an additional feature in the asymmetric cam **430** is seen as a diminution in the thickness along three faces **431** of the asymmetric cam **430** which is a relief in the rear of the asymmetric cam **430.** The significance of this relief **431** is that it restricts the contact of the implant **410** with the shoulder **405** to the extent defined by the relief in the rear of the asymmetric cam **430.** As noted above, in fashioning an implant site in the intervertebral space during a partial discectomy, surgeons are unable to produce perfectly sharp angles. To accommodate this imperfection, to prevent trauma upon insertion of an implant with sharp edges, and to create as tight-fitting an implant as possible, the fashioning of a bevel around the edges of the implant that are inserted into the intervertebral space created by the surgeon is desired. At the same time, in order to prevent backing out of the implant, it may be desirable to retain a sharp anterior implant edge, and therefore the relief in the cam **430** does not extend completely around the cam. Thus, upon completion of the external profile of the implant **410** as described above, the carriage **442** is backed away from the cutter, the stop cam **490** is flipped to its second position allowing advancement of the stop screw **470** the additional approximately 0.06" mentioned above. At the same time, a shot pin **432** is advanced into the relief **431** by means of a shot pin mover **433,** thereby allowing rotation of the cam **430** only to the extent permitted by the shot pin **432** as it rides within the relief **431.** With the shot pin **432** riding in the relief **431,** the "shoulder" **405** contacts the leading edge **411** of the implant blank **410,** thereby rounding three edges of the implant **410.** After machining the leading edge **411** of the implant **410,** the implant is removed from the spindle **420,** turned around, and re-positioned on the spindle **420,** to inscribe the bevel on three edges of the other side of the implant.

In figure 4D, a frontal view is provided of the spindle **420,** the implant **410,** the asymmetric cam **430,** and the cam follower **460.** Also shown is the cam adapter **461,** by means of which the cam follower **460** is affixed to the carriage **442,** and by means of which the cam follower **460** maintains the cutting surfaces **404/405** in contact with the implant **410** as defined by the shape of the asymmetric cam **430.** Also shown is a part of the cross-slide **441,** which is preferably biased or which may be pushed manually toward the cam follower **460.**

In figure 4E, a side detail view is provided of the stop cam **490.** In this view, a stop cam handle **491** is shown which allows the operator of the implant outside profile generator to fix the stop cam **490** in a first position **A,** and a second position **B,** whereby additional travel of the stop screw **470,** and thereby advancement of the carriage **442,** is provided in position **B,** of about 0.06" due to the difference in the distances shown for these positions.

By means of the apparatuses and method described above, a cortical bone implant **100** as shown in figure 1 having a substantially "D"-shaped external profile, and a substantially "D"-shaped internal canal is produced. Naturally, based on this disclosure, those skilled in the art will appreciate that other shapes, both for the external profile and internal canal of the implant may be produced. For example, an ellipsoid is produced by the above described methods simply by modification of the shape of the asymmetrically shaped cam **430,** and the internal canal shape may be modified by drilling, routing, or broaching using a broach that tapers to any desired shape. The thus formed implant may be used after machining as described, followed by appropriate cleaning methods known in the art (e.g. bathing in alcohol, peroxide treatment etc.). In addition, however, it may be desirable to inscribe an external feature on the upper surface **110,** the lower surface **111,** or both. Such a feature may take any desirable form, such as annular rings, indentations, projections, ribbing or teeth. In a preferred embodiment, teeth sloping toward the anterior aspect **108** of the implant are inscribed onto the top **110** and bottom **111** surfaces of the implant by forcing the implant through opposed broaches bearing inscribing teeth. Alternatively, the upper **110,** lower **111** or both surfaces in turn may be repeatedly run, manually or by a machine-driven means, over an appropriately fashioned jaw bearing abrasive teeth such that the required profile of teeth are inscribed into the surfaces of the implant. Desirably, the successive teeth of the jaw are incrementally raised in height such that each tooth is only required to remove a small amount of bone (about 0.0016 cms (0.004") per tooth, to a total depth of 0.006 cms (0.015"). In addition, it is preferred that the rake (angle of the teeth) be sufficiently sharp as to allow the implant to bite into the implantation site, without at the same time being so sharp as to be excessively brittle.

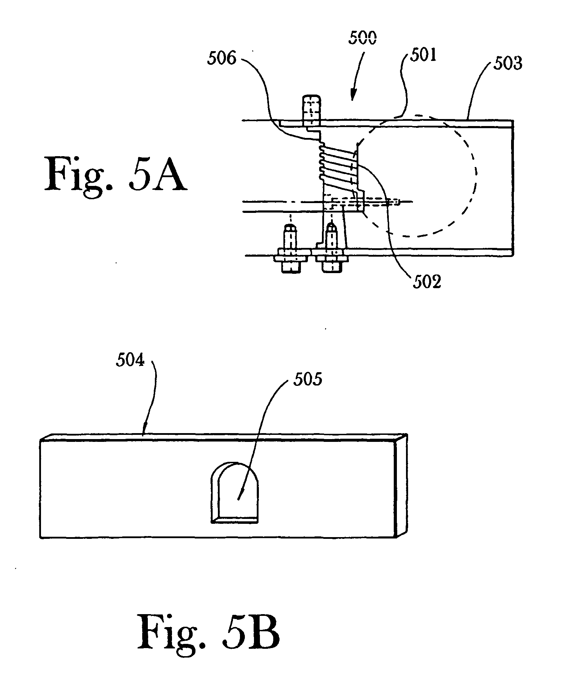

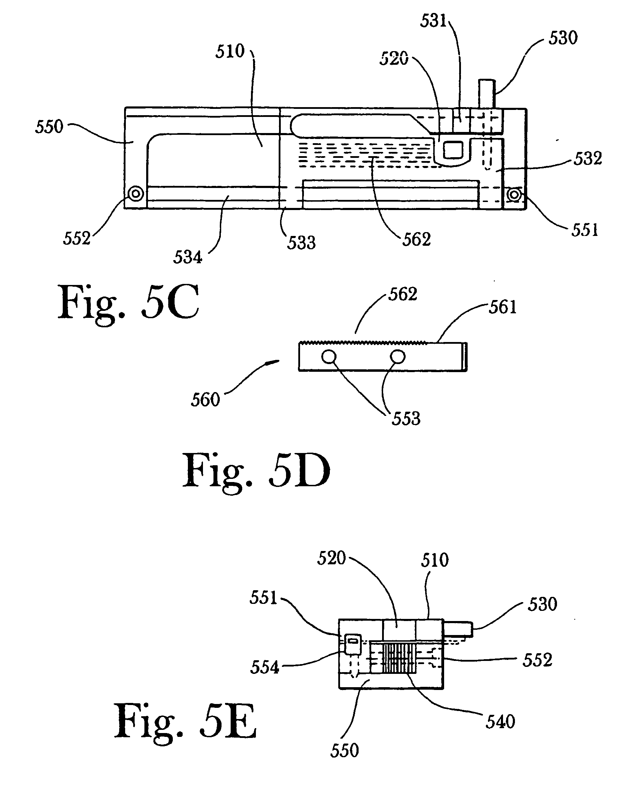

In figure 5, figure 5A, there is provided a top view of one side of blades **502** for use in a broach assembly 500 for inscribing teeth into the top **110,** bottom 111 or both surfaces of the implant. In outline, there is shown a lock-down handle **501** for clamping the assembly of blades **502** to a base **503.** By bringing a mirror image jaw into register with the depicted broach, a space is formed between the opposing teeth **502** at a distance sufficient to accommodate passage of the implant therebetween, provided that the teeth abrade recesses into the top and bottom surfaces of the implant **100.** To ensure proper engagement of the blades **502** and the implant **100,** there is provided a non-cutting surface **506** for contacting the implant **100** as it is introduced into the broach assembly **500.** The non-cutting surface **506** acts as a type of micrometer, forcing the cutting surfaces of the teeth **502** sufficiently apart to properly engage the implant as it passes through the broach assembly **500.** In figure 5B, there is provided a side view of an implant mounting device **504** having a "D"-shaped cavity 505 into which a "D"-shape implant may be fitted for passage through the opposing jaws of the broaching jaw apparatus **500.** The resultant implant has the profile shown in figures 1C-1E.

In figures 5C-5E, there is shown an alternate apparatus and method for fashioning the retention teeth in the implant. In figure 5C, there is shown a carriage **510** having an appropriately dimensioned slot **520** for receiving the implant to be grooved. A tensioning screw **530** brings a retention arm **531** into juxtaposition with carriage housing member **532,** thereby clamping the implant into position within slot **520.** Through carriage housing members **532** and **533,** there is aligned a guide-rod **534** for guiding the carriage containing the implant as it is raked across a blade assembly **540,** over which said carriage **510** is made to pass. Said guide rod **540** also conveniently acts as a hinge, allowing the carriage **510** to swing upward for implant loading and also permitting the carriage to move down toward the base as the implant surface is cut on each successive pass of the carriage over said blade assembly **540.** The blade assembly **540** is bolted within a base **550** over which said carriage **510** slides. Said base **550** also acts to receive fixation screws **551** and **552** which retain said guide rod **534** in place. A plurality of individual blades **560** are placed in a recess **554** in the base **550** and are maintained in registered position by retention screws **552** passing through retention holes **553** in each blade. Each blade **560** has an initial non-cutting surface **561,** which is approximately 0.015" below the cutting surface **562,** which in combination with said plurality of blades, forms a flat loading area for implant insertion into said slot **520.** Figure 5D provides a side view of one blade **560,** while figure 5E provides an end on view of the carriage **510** as it sits above the base **550.** Accordingly, the implant is inserted into the slot **520** with the carriage **510** swung up from the base **550.** The carriage is then swung down into the starting position with the implant making contact with the non-cutting surfaces **561** of the plurality of blades. The implant is depressed so that it is forced snugly against the non-cutting surface, and then tensioned into place with the retention screw **530.** Thereafter, the carriage is slid several times over the base **550** such that the cutting surfaces **562** of the plurality of blades thereby inscribe the desired tooth structure into the top surface, the bottom surfaces or both (after switching the implant around) surfaces of the implant. When the metallic bottom of the carriage comes into contact with the base, the machining of the implant is complete.

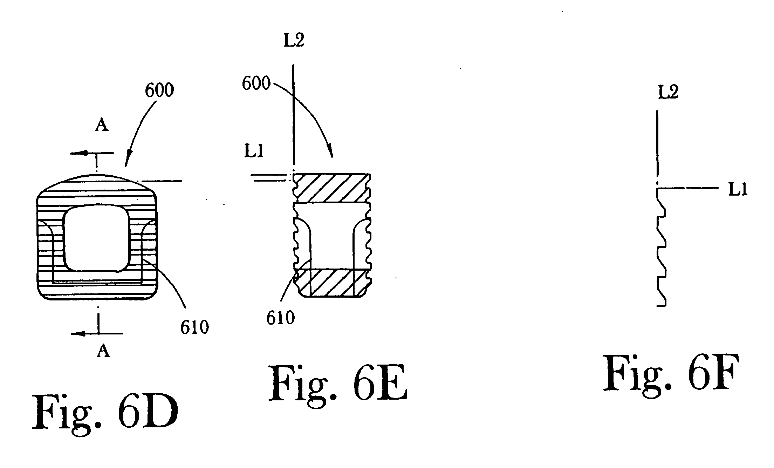

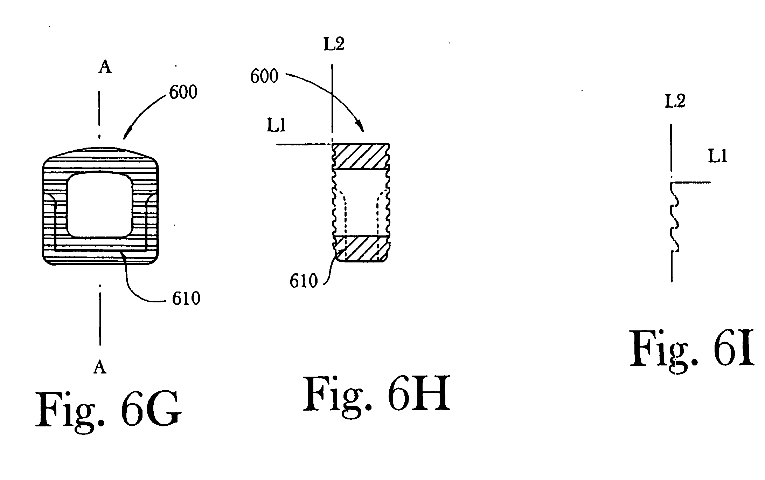

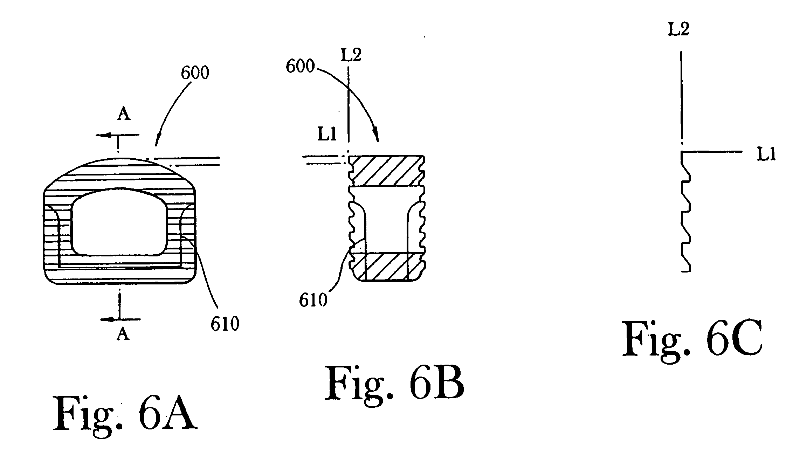

In figure 6A-I, there is provided a view of three different cortical bone implants not according to this invention having particular geometries by way of example. In figure 6A, there is shown an example of an implant **600** which has a height of 7 mm, a width of 11 mm, and a length of 14 mm. In addition, dimensions of various radii of the implant are provided. Note the effect of the "shoulder" **405** of the cutter which produces the a 0.23mm (0.059") radius and indent profile **610** starting at the approximate center of the part and proceeding around to the opposite side of the implant, i.e. around three faces of the implant. In figure 6B, the implant **600** is shown as a side view, and in figure 6C, there is shown a detail view of the teeth. Identical descriptions apply to the 7mmX11mmx11mm views of the implants of figures 6D-6F and the 7mmX14mmx14mm implant of figures 6G-6I.

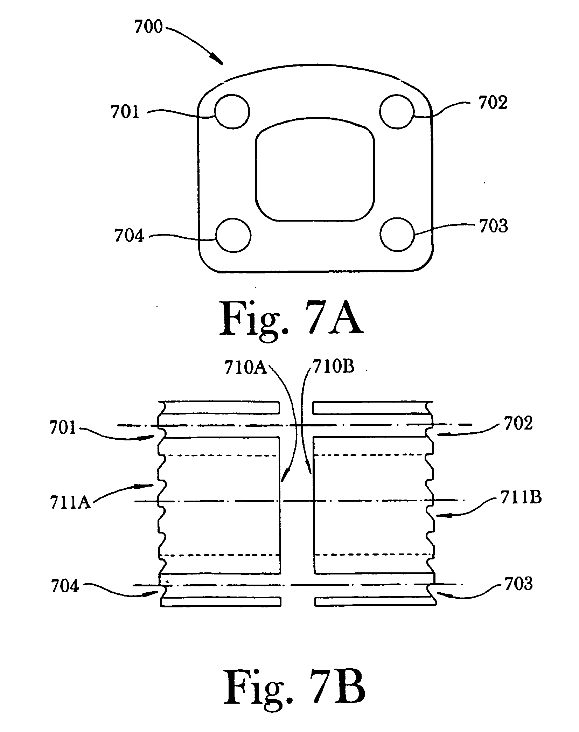

In figure 7, there is shown an implant, either machined as described above, or prior to said machining, which is further machined so as to allow stacking thereof to achieve implants of various heights. Commencing from a blank cortical plug at the stage shown in figure 2D has the advantage that if breakage of the implant occurs during machining, this will likely occur prior to completion of all of machining steps. Two implant blanks of known height are selected such that a unitary implant composed of both starting implants can be produced of a new desired height (e.g. a 6 mm high implant may be stacked with a 7 mm high implant to produce a 13 mm implant). Each implant blank is placed in a drill jig, and by means of a drill press or like means, holes are drilled through the implants. With the implants still in the jig, the jig is placed on the table of an arbor press. Pins, composed of cortical bone, resorbable but strong biocompatible synthetic material, or metallic pins of the appropriate diameter are then impelled into the holes in the implants such that the implants are formed into a unitary body by these pins. In order to encourage bony ingrowth, channels may be cut into the adjacent surfaces of the implants. The embodiment shown in figure 7A is a top view of an implant **700** into which four holes **701-704** have been drilled. In figure 7B, there is shown the juxtaposition of two implants **700A** and 700B, with the drilled holes **701-704** in register to receive pins for maintaining the implants in register. In this view, the adjacent surfaces **710A** and **710B** have not been inscribed with teeth, while the surfaces **711A** and **711B** have been so inscribed. Based on this disclosure, those skilled in the art will recognize that a number of variations and modifications may be made to stack various forms of bone implants, or to maintain such implants in register with each other. Thus, as shown in figure 9, an implant **900** is produced by producing two implants **901** and **902,** each having a cavity or canal **903,** including an asymmetric key way **904** machined therein. By press-fitting the two implants together using an appropriately shaped cancellous plug **905** or a plug made from another biocompatible material, including but not limited to hydroxyapatite, cortical bone, synthetic materials, ceramic, optionally treated with growth factors such as bone morphogenetic protein and the like, the two implants **901** and **902** are retained in registered juxtaposition to form the implant **900.**

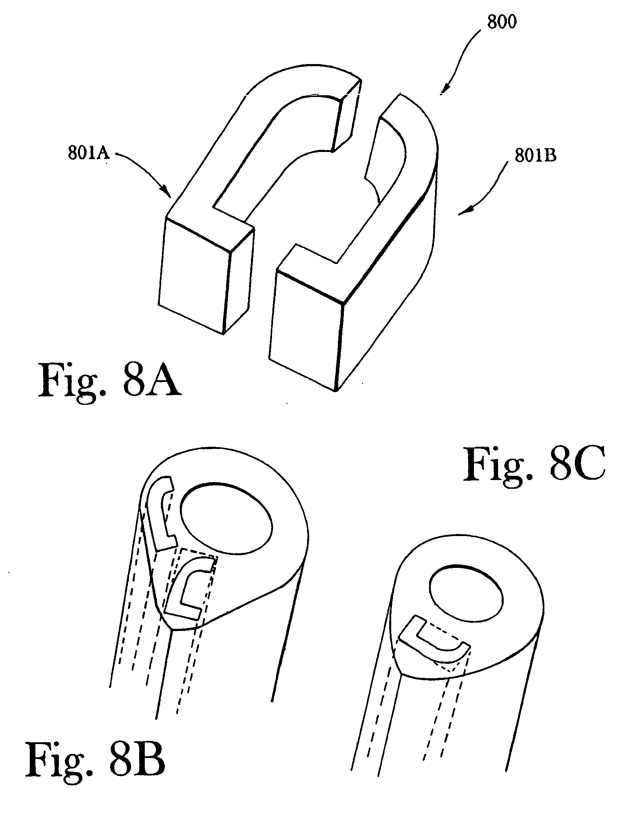

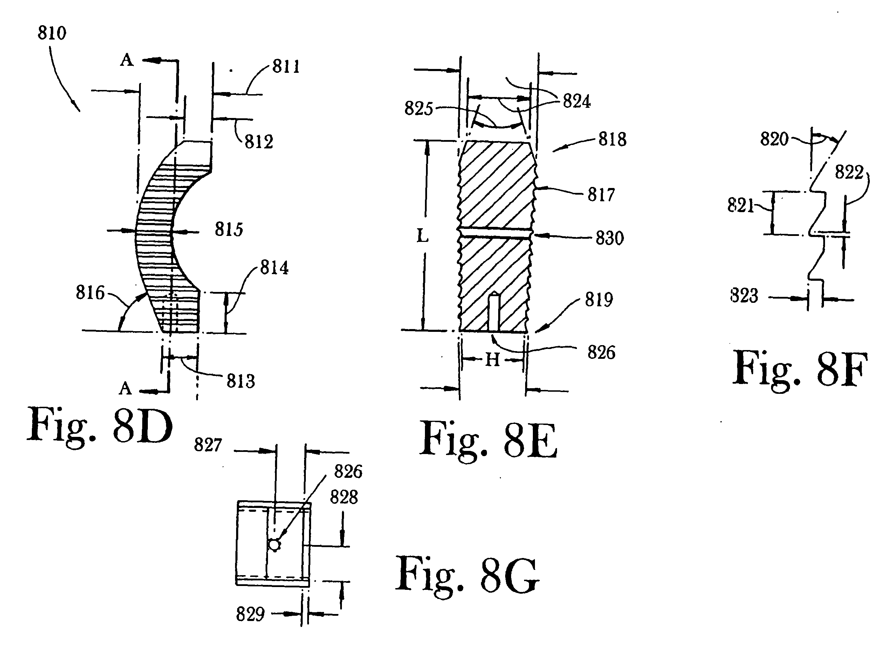

In an embodiment of this invention, shown in figure 8, a method for assembling implants from component parts is provided. In figure 8A, there is shown an implant **800** composed of two side-by-side halves, **801A** and **801B.** The two halves of the implant are brought into juxtaposition to form a unitary implant. The two halves may be implanted in juxtaposition, or holes may be formed in each half, and the halves maintained in contact by forcing pins through the holes, in a fashion analogous to that described above for maintaining stacked implants in contact with each other. For this embodiment of the invention, a portion of cortical bone may be harvested from any suitable source of cortical bone. As shown in figure 8B, a segment, in the form of a block or a column of cortical bone is harvested along the long axis of a long bone, such as the femur, tibia, or fibula. The shape of the bone may be inscribed into the thus-harvested cortical bone by routing, broaching or other means as described herein. The thus-machined cortical bone may then be sectioned into appropriate heights, as needed, to provide the implant halves **801A** and **801B.** Alternate sites for harvesting the cortical bone segment are shown in figures 8B and 8C.

In addition to use for cervical Smith-Robinson type fusion, implants comprising each element, **801A** or **801B** alone, modifications and variations thereof, optionally in combination with another vertebral fusion implant, may be implanted, for example, to assist in induction of posterior lumbar intervertebral fusion (PLIF). In such a case, a device **810,** such as that shown in figure 8D-8G is machined from bone stock as shown in figures 8B, 8C or another appropriate bone stock, and is inserted, according to methods known in the art for insertion of PLIF implants. Preferably, the device as used for FLIP applications has the following dimensions similar to the following, see side view figure 8D:a width **811** of approximately 7 to 12 mm, and preferably about 9.4 to about 10 mm; a top dimension **812** of about 4 to 5 mm; a bottom dimension **813** of about 4-6 mm and preferably about 5 mm; a flat surface of **814** of about 4-7 mm, and preferably about 5.5 mm; a width **815** of about 5-7 mm and preferably about 5 mm; a curvature that defines an angle **816** of between about 60 and 75 degrees, and preferably about 67 degrees. See figure 8E, rear view: a length L of about 20 to 26 mm; a height **H** of between about 7.5 and 14.5 mm; preferably, heights of about 8, 10, 12, and 14 mm are produced with lengths of about 20 and 26 mm; desirably, the side faces **817** are machined to display a rough, ridged or grooved surface so that when the anterior end **818** of the PLIF implant is properly seated in place, ridges directed to the posterior end **819** of the PLIF implant prevent backing out of the implant. A detail of one embodiment of such a ridged surface is shown in figure 8F, wherein the following dimensions are preferred: an angle **820** for each tooth of between about 30 and 40 degrees, preferably about 35 degrees; a distance between tooth crests **821** of about 1-2 mm, preferably about 1.5 mm; a tooth crest width **822** of about 0.1 to about 0.2 mm, preferably about 0.125 mm; and a tooth height **823** of between about 0.1 to about 1 mm and preferably about 0.5 mm; returning to figure 8E, the implant preferably has an anterior end width **824** of about 7-13 mm, preferably about 9-13 mm, with a taper angle **825** from the height H of about 30 to 40 degrees, preferably about 35 degrees; an instrument attachment means, **826,** such as a tapped instrument attachment hole, is provided in the posterior face of the PLIF implant; this feature is best seen from the posterior view 8G, which shows: an instrument attachment hole **826** having a diameter of about 1.5 to about 2.5 mm, preferably about 2 mm, and a depth of about 4-5 mm, preferably about 4.5 mm; an edge to center of the instrument attachment hole dimension **827** is carefully defined to match dimensions of any implant insertion device used in combination with this embodiment of the PLIF implant; a center of the instrument attachment hole to edge dimension **828** is about 4-6 mm, preferably about 5 mm, with a ridge **829** of about 1 mm running along three edges of the posterior face of the implant. In displaying the section A-A from figure 8D in figure 8E, a slight air gap **830** is shown as the section would exit bone on the concave surface of the implant and then reenter bone.

In use, the implant **810** is inserted on either side of lumbar intervertebral spaces to thereby stabilize and assist in fusion of adjacent lumbar vertebrae. This is accomplished by distraction of the lumbar vertebrae, removal of an appropriate amount and shape of intervertebral disc matter, and insertion of the implant **810,** preferably on each side on a posterior approach, according to methods known in the art. The concave surface of each implant **810** is set to face inwardly, toward the center of the vertebral body, while the convex surface of the implant **810** is set to match, as much as possible, the natural external curvature of the lumbar vertebrae.

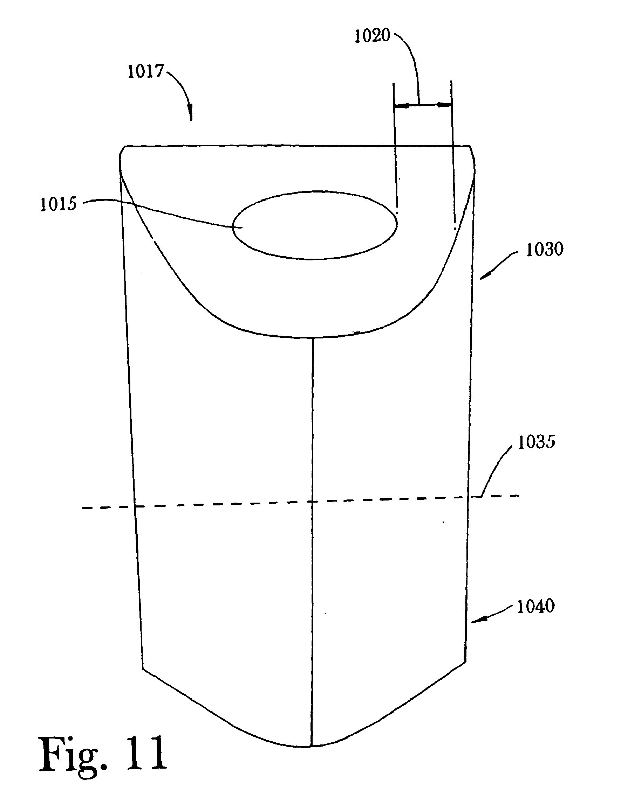

In an analogous but alternate method for production of the cervical implant, unitary implants may be produced by appropriately sectioning and machining the anterior margin of the tibia or linea aspera of the femur. Thus, as shown in figure 10A, a left femur **1000** (posterior aspect), or in figure 10B, a left tibia **1001** (anterior aspect), is sectioned at **1004** and **1005** to remove the head, neck and greater trochanter **1002** and internal and internal condyles **1006** of the femur, or tubercle and tuberosity **1003** malleolus **1007** of the tibia. The result from such sectioning is the production of a shaft, or diaphysis, of the femur **1008** or tibia **1009.** Further processing according to this aspect of the invention involves the line asper **1010** of the femur or the anterior margin of the tibia **1011,** as shown in figure 10C. Whether produced from the femur or tibia, a diaphysial shaft **1012,** extending as shown at **1016** to a length permitted by the length of the shaft produced by the sectioning at **1004/1005.** The shaft comprises the natural intramedullary canal **1013.** The thus produced shaft is then further sectioned in a plane shown at **1014** to produce a shaft of bone removed from the natural intramedullary canal **1013** having a cylindrical but somewhat triangular external shape. Into this shaft may be drilled a cannulation **1015,** as shown in figure 11.

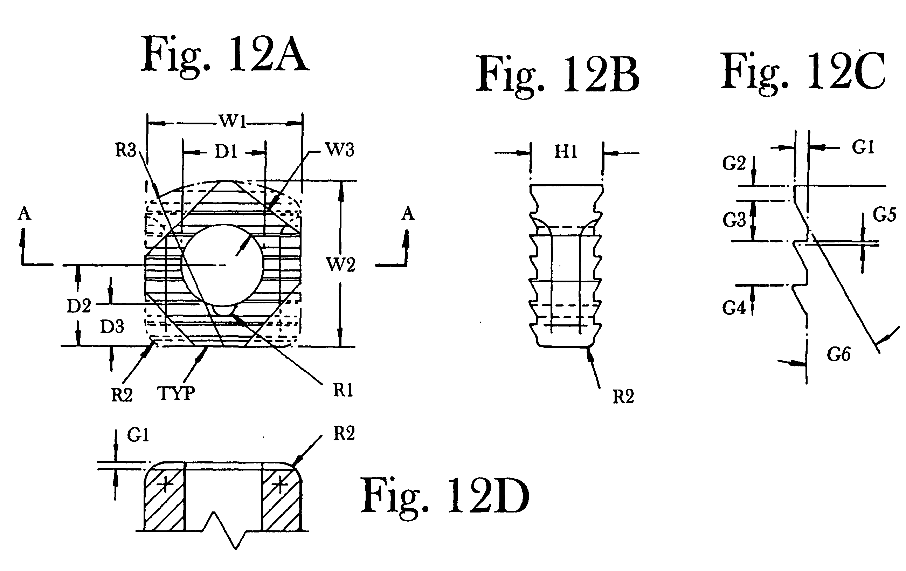

Figure 11 shows the substantially triangular shaft of substantially cortical bone **1017** produced by sectioning the shaft of the long bone down the plane **1014.** Into the shaft of bone **1017** may be drilled a bore to produce a cannulation **1015** of appropriate dimensions. The cannulation **1015** may be introduced into the unitary shaft of bone **1017** or it may be introduced into sub-segments thereof by first cutting the shaft **1017** at **1035.** In either case, the diameter of the cannulation **1015** should be limited such that at the narrowest portion between the cannulation and the wall of the device **1020** never falls below about 2 mm. When sectioned at **1035,** for example by mounting the shaft **1017** on a lathe and contacting the spinning shaft with a very narrow blade (i.e. a parting tool of about 1 mm or less width), implant blanks 1030 and **1040** are produced which may be further machined to achieve desired external and internal profiles and key way features, as described for the implant of this invention produced by alternate methods described hereinabove. Implants of any desired height, for example 5 mm to about 14 mm, may thus be produced. Figures 12-17 show specific embodiments of implants produced according to this method.

Per figures 12-17, there is provided views of five different cortical bone implants having particular geometries. In each figure, view A is a top view, view B is a side view, view C is a detail of the grooves which angle toward the posterior aspect of the implant, and view D is a sectional view through the line A-A shown in view A. In addition, where an osteogenic plug, such as a cancellous plug is present, this is shown in view E as a top view and view F as a side view of the cancellous plug. In figure 12, there is shown an example of an implant having a height **H1** between about 5mm and about 9mm, a width **W1** of about 11 mm, and a width **W2** of about 11 mm. An outer dotted profile provides a means for comparing the shape of the implant produced according to this alternate manufacturing method with the external profile of the implant of figure 6. As can be seen, the implant produced has a substantially diamond-shaped external profile, as a result of the geometry of the starting shaft **1017** of bone stock.

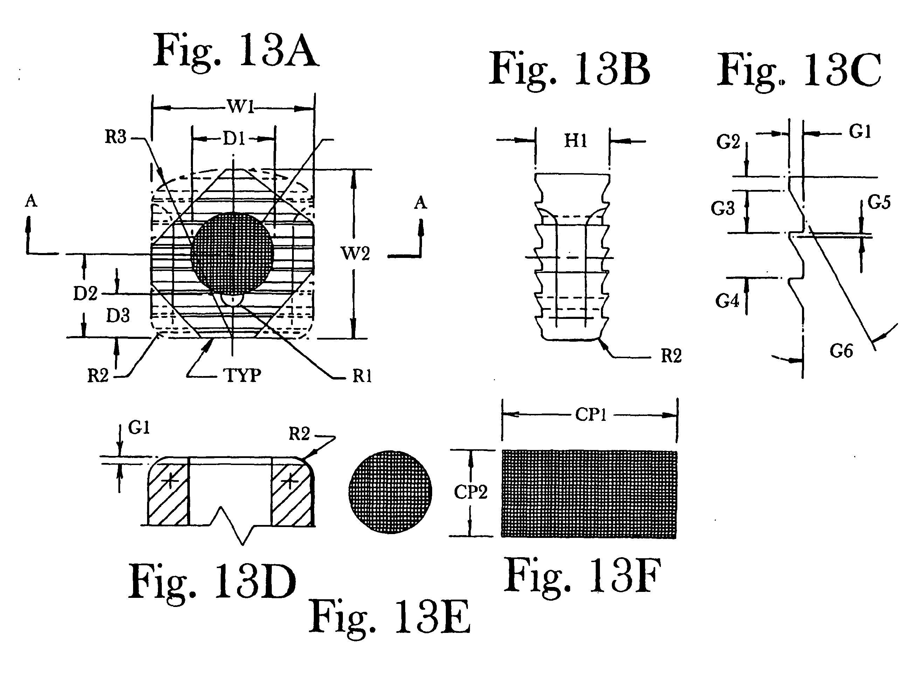

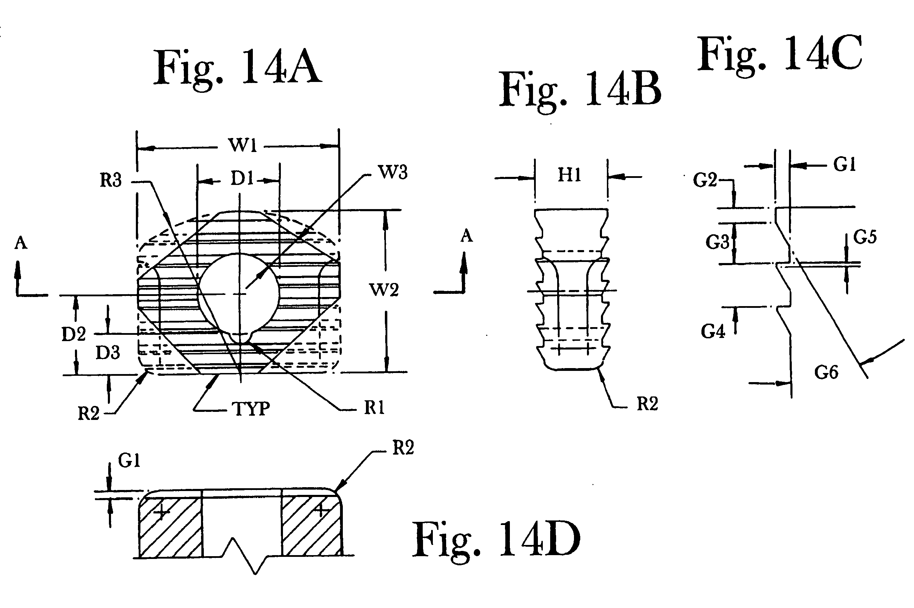

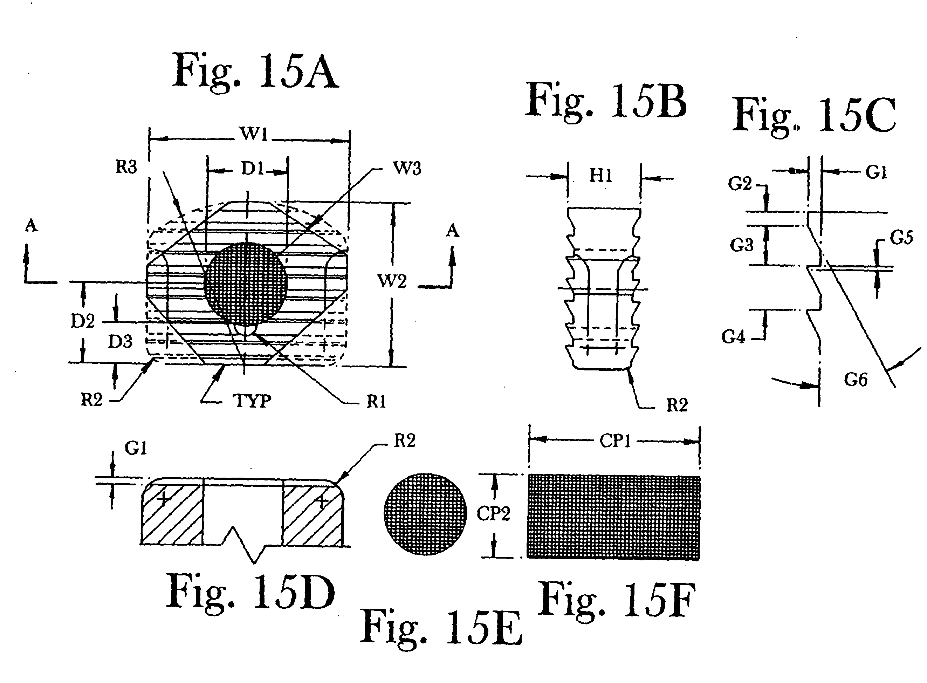

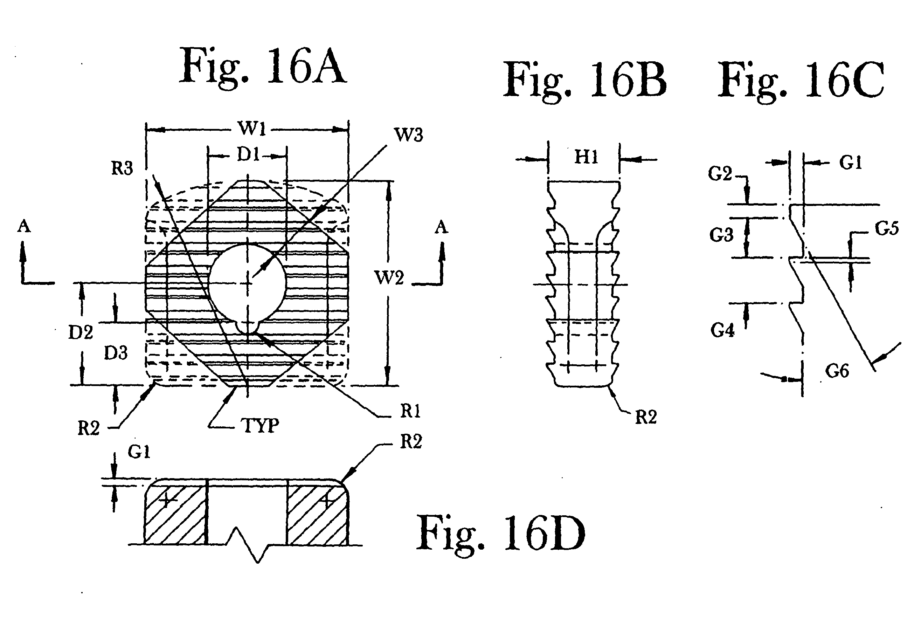

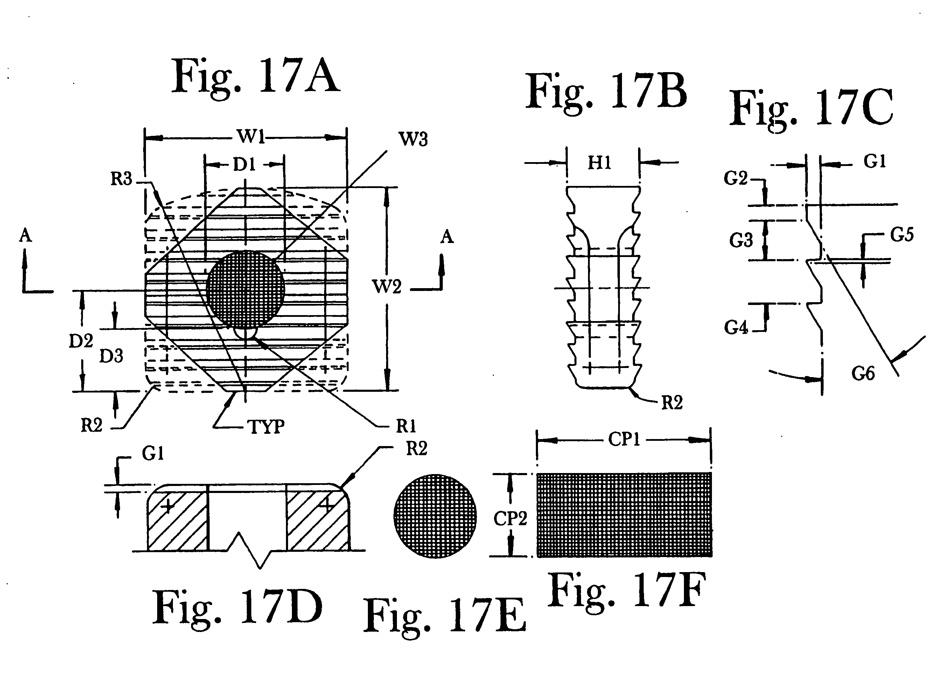

Figure 13 shows a device similar to that of figure 12, with a cancellous plug inserted therein. Figure 14 shows a device having a width **W1** of about 14 mm and a height **H1** of between about 5 mm and about 14 mm. Figure 15 shows a device similar to that of figure 14 with a cancellous plug inserted therein. Figure 16 shows a device having a width **W1** of about 14 mm, a width **W2** of about 14 mm, and a height of between about 5 mm and 11 mm. Figure 17 shows a device similar to that of figure 16 having a cancellous plug inserted therein. Table I below summarizes the various features and provides examples of specific dimensions for various embodiments of the implant shown in figures 12-17:

**Table I:**

| | | | | | | | |
|---|---|---|---|---|---|---|---|
| | | **Figure #** | | | | | |

| Code | Description | 12 | 13 | 14 | 15 | 16 | 17 |
|---|---|---|---|---|---|---|---|
| D1 | Inner Hole Diameter | 5.5 | 5.5 | 5.5 | 5.5 | 5.5 | 5.5 |
| D2 | Hole Centerline Distance | 5.5 | 5.5 | 5.5 | 5.5 | 5.5 | 5.5 |
| D3 | Key Way Centerline Distance | 2.9 | 2.9 | 2.9 | 2.9 | 4.4 | 4.4 |
| G1 | Groove Depth | 0.5 | 0.5 | 0.5 | 0.5 | 0.5 | 0.5 |
| G2 | Top Flat Width | 0.5 | 0.5 | 0.5 | 0.5 | 0.5 | 0.5 |
| G3 | Groove Pitch width without G5 | 1.375 | 1.375 | 1.375 | 1.375 | 1.375 | 1.375 |
| G4 | Groove Pitch width with G5 | 1.5 | 1.5 | 1.5 | 1.5 | 1.5 | 1.5 |
| G5 | Flat Width | 0.125 | 0.125 | 0.125 | 0.125 | 0.125 | 0.125 |
| G6 | Groove Angle (degrees) | 30 | 30 | 30 | 30 | 30 | 30 |
| H1 | Overall Height | 5-9 | 5-9 | 5-13 | 5-13 | 5-11 | 5-11 |
| R1 | Keyway Radius | 0.8 | 0.8 | 0.8 | 0.8 | 0.8 | 0.8 |
| R2 | Corner Radius | 1.5 | 1.5 | 1.5 | 1.5 | 1.5 | 1.5 |
| R3 | Outside Arch Radius | 11 | 11 | 11 | 11 | 14 | 14 |
| W1 | Outside Width | 11 | 11 | 14 | 14 | 14 | 14 |
| W2 | Outside Width | 11 | 11 | 11 | 11 | 14 | 14 |
| W3 | Wall Thickness | ≥2 | ≥2 | ≥2 | ≥2 | ≥2 | ≥2 |
| CP1 | Cancellous Plug Height | N/A | 5-9 | N/A | 5-13 | N/A | 5-11 |
| CP2 | Cancellous Plug Diameter | N/A | 5.7-5.8 | N/A | 5.7-5.8 | N/A | 5.7-5.8 |

### 4.4 Manner of Using the Implant

In use, the implant **100** is inserted into a space formed between adjacent vertebrae that are required to be fused. This may be accomplished by the surgeon removing portions of the intervertebral disk, (partial discectomy) and retracting the adjacent vertebrae to allow insertion of an appropriately dimensioned implant. The rear end **105** of the implant is inserted first, and where present, the external feature **120** prevents backing out of the implant. Where no external feature **120** has been inscribed into the top and bottom surfaces of the implant, it may be necessary to affix the implant in position with plate and screw retention systems known in the art. According to this invention, implants are provided having a height of between about 7 and 14 mm, a length of between about 11 and 14 mm and a width of between about 11 and 14 mm. Any permutation or combination of these dimensions may be envisioned, for example (in order of height, length, width): 7x11x11, 8x11x11, etc.; 7x14x14, 8x14x14, etc.; 7x11x14, 8x11x14, etc.

Preferably, the surgeon performing the implantation saves the autologous material and debris produced in the course of the partial discectomy for packing into the canal of the present implant. In addition, or alternatively, the canal may be packed (either during the surgical procedure or the canal may be pre-packed) with osteogenic, osteoinductive, or osteoconductive materials, including but not limited to: allograft bone, autograft bone, autogenous osteogenic materials including bone marrow cancellous bone and the like, demineralized bone, freeze-dried demineralized bone, Grafton® (demineralized bone in glycerol), bone powder, bone derivatives, bone morphogenetic protein (purified or recombinant), antibiotic, bioactive glass, hyrdorxyapatite, bioactive ceramics, or combinations thereof.

Following implantation, the recipient (whether human or animal) is monitored for implant stability and success in fusion. Fusion is achieved over the course of several weeks to several months, during which time increasing levels of load may be placed on the spine.

### 5.0 References

Bailey, R.W., and Badgley, L.E. (1960) Stabilization of the Cervical Spine by Anterior Fusion, J. Bone and Joint Surg. 42A:565-594.
Cloward, R. B. (1958) The Anterior Approach for Removal of Ruptured Cervical Discs, J. Bone and Joint Surg. 15:602-617.
Grooms et al., (1996) Success of Surgery on the Anterior Cervical Spine: Smith-Robinson Technique vs. Internal Plates, Clinical Performance of Skeletal Prostheses, L.L. Hench and J. Wilson, Eds., Chapman & Hall.
Robinson, R.A., et al., (1962) The Results of Anterior Interbody Fusion of the Cervical Spine, J. Bone and Joint Surg. 44A:1569-1587.
Robinson, R.A. and Smith, G.W. (1955) Anterolateral Cervical Disc Removal and Interbody Fusion for Cervical Disc Syndrome, Bull. Johns Hopkins Hosp. 96:223-224.
Smith, G.W. and Robinson, R.A., (1958), The Treatment of Certain Cervical-Spine Disorders by Anterior Removal of the Intervertebral Disc and Interbody Fusion, J. Bone and Joint Surg. 40A:607-623.
White, A.A. III, and Hirsh, C. (1972) An Experimental Study of the Immediate Load Bearing Capacity of Some Commonly Used Iliac Bone Grafts, Acta Orthop. Scandanav. 42:482-490.
Whitecloud, T.S. III and Dunsker, S. (1993) Anterior Cervical Spine Surgery, Principles and Techniques in Spine Surgery, Raven Press, N.Y.
U.S. Patent No. 5,306,309
U.S. Patent No. 5,609,635
U.S. Patent No. 5,306,307
U.S. Patent No. 4,950,296

## Claims

1. At least one implant consisting substantially of cortical bone, said implant comprising an open canal surrounded by a continuous wall of cortical bone in a half "D"-shape, wherein said open canal has a half "D"-shape, thereby forming an implant having a top face and a bottom face, each of which is substantially planar, with said planes being substantially parallel to each other.

2. The implant of claim 1 further having an external feature on said top face, said bottom face or both.

3. The implant of claim 2 wherein said external feature is at least one groove or ridge.

4. The implant of claim 3 wherein said external feature is a series of ridges.

5. A stack of at least two implants of claim 1.

6. The stack of claim 5 wherein said stacked implants are pinned to each other.

## Patentansprüche

1. Mindestens ein Implantat, das im Wesentlichen aus Knochenrinde besteht, wobei das Implantat einen offenen Kanal umfasst, der in einer halben ,D'-Form von einer fortlaufenden Wand aus Knochenrinde umgeben ist, wobei der offene Kanal eine halbe ,D'-Form aufweist, wobei dieser ein Implantat bildet, das eine obere Fläche und eine untere Fläche hat, von denen jede im Wesentlichen eben ist, und wobei die Ebenen im Wesentlichen parallel zueinander liegen.

2. Das Implantat gemäß Anspruch 1, das weiterhin ein äußeres Merkmal auf der oberen Fläche, unteren Fläche oder beiden aufweist.

3. Das Implantat gemäß Anspruch 2, wobei das äußere Merkmal mindestens eine Kerbe oder ein Grat ist.

4. Das Implantat gemäß Anspruch 3, wobei das äußere Merkmal eine Reihe von Graten ist.

5. Ein Stapel von mindestens zwei Implantaten gemäß Anspruch 1.

6. Ein Stapel gemäß Anspruch 5, wobei die gestapelten Implantate miteinaner verstiftet sind.

## Revendications

1. Au moins un implant constitué substantiellement d'os cortical, ledit implant comprenant un canal ouvert entouré d'une paroi continue d'os cortical en forme d'un demi « D », dans lequel ledit canal ouvert a une forme en demi « D », formant ainsi un implant ayant une face supérieure et une face inférieure, dont chacune est substantiellement plane, lesdits plans étant substantiellement parallèles l'un par rapport à l'autre.

2. Implant selon la revendication 1 présentant, en outre, une particularité externe sur ladite face supérieure, ladite face inférieure ou sur les deux.

3. Implant selon la revendication 2, dans lequel ladite particularité externe est au moins un sillon ou une crête.

4. Implant selon la revendication 3, dans lequel ladite particularité externe est une série de crêtes.

5. Pile d'au moins deux implants selon la revendication 1.

6. Pile selon la revendication 5, dans laquelle lesdits implants empilés sont chevillés les uns aux autres.
